(19) Europäisches Patentamt
European Patent Office
Office européen des brevets

(11) **EP 2 271 256 B1**

(12) **EUROPEAN PATENT SPECIFICATION**

(45) Date of publication and mention
of the grant of the patent:
**01.08.2012 Bulletin 2012/31**

(51) Int Cl.:
*A61B 5/053* (2006.01)   *A61B 5/107* (2006.01)
*A61B 5/05* (2006.01)

(21) Application number: **09726147.3**

(22) Date of filing: **26.03.2009**

(86) International application number:
**PCT/IB2009/051255**

(87) International publication number:
**WO 2009/118701 (01.10.2009 Gazette 2009/40)**

(54) **METHOD AND SYSTEM FOR MEASURING AN OBJECT OF INTEREST**

VERFAHREN UND SYSTEM ZUR MESSUNG EINES BESTIMMTEN OBJEKTS

PROCEDE ET SYSTEME DE MESURE D UN OBJET D INTERET

(84) Designated Contracting States:
**AT BE BG CH CY CZ DE DK EE ES FI FR GB GR HR HU IE IS IT LI LT LU LV MC MK MT NL NO PL PT RO SE SI SK TR**

(30) Priority: **27.03.2008 CN 200810090345**

(43) Date of publication of application:
**12.01.2011 Bulletin 2011/02**

(73) Proprietor: **Koninklijke Philips Electronics N.V.
5621 BA Eindhoven (NL)**

(72) Inventors:
• **YAN, Ming
Shanghai 200233 (CN)**
• **JIN, Hua
Shanghai 200233 (CN)**
• **ZHANG, Xin
Shanghai 200233 (CN)**

(74) Representative: **Kroeze, Johannes Antonius
Philips
Intellectual Property & Standards
P.O. Box 220
5600 AE Eindhoven (NL)**

(56) References cited:
EP-A- 1 767 147       WO-A-01/87415
WO-A-2007/040494    WO-A-2007/128952
JP-A- 2 031 736       JP-A- 2 031 739

EP 2 271 256 B1

**Description**

FIELD OF THE INVENTION

The invention relates to a method and system for measuring an object of interest, for example in an imaging system, such as an electrical impedance tomography system or a magnetic induction tomography system.

BACKGROUND OF THE INVENTION

Electrical impedance tomography (EIT), also referred to as applied potential tomography (APT), is an imaging technique that is particular used in medical applications. This technique images the spatial distribution of electrical impedance inside an object, such as the human body. The technique is attractive as a medical monitoring tool because it is non-invasive and does not use ionizing radiation, as in X-ray tomography, or the generation of strong, highly uniform magnetic fields, as in Magnetic Resonance Imaging (MRI), and can thus be used in bed-side long term *in vivo* monitoring.

Prior art document WO2007/128952A1 discloses an apparatus for electrical impedance imaging of an object of interest that comprises first and second electrode arrangements spaced apart to define an imaging region therebetween. An object to be imaged is locatable, in use, in the imaging region so that impedance data can be collected from the object using the first and second electrode arrangements to permit the construction of an impedance image of the object.

However, as EIT electrodes are attached to the patient body, when applying EIT in a long term *in vivo* monitoring, changes of the patient body position may affect electrode positioning, which may introduces errors in measurement data and thus introduces artefacts into the images reconstructed from the measurement data. Furthermore, measurement electrodes are also difficult to be positioned accurately because of the irregular geometry of the patient body. Therefore, it is difficult to get precise electrodes position information with current configuration. The errors of electrode positions are main contributors to the errors in the reconstruction procedure.

Obtaining the precise positioning information of measurement sensors, and thus the positioning of the object of interest to be imaged is also helpful for imaging reconstructions in other imaging systems. For example, when applying magnetic induction tomography (MIT) system in patient monitoring, the position and shape information of the object of interest can be used for determining the imaging reconstruction region that leads to improve the image quality.

Prior art documents JP 02 031736 A and JP 02 031739 A disclose a helmet having a plurality of spring-loaded rods on its interior side. When put onto the cranium of a patient, the rods are pushed into the helmet to an extend that is measured via a resulting resistance variation. Moreover, electrical potentials at the surface of the head can be recorded via the rods.

SUMMARY OF THE INVENTION

An object of the invention is to develop an alternative system of measuring an object of interest that can help derive the position and/or the shape of the object of interest.

To this end, this invention provides a system of measuring an object of interest, comprising:

-    a frame having a plurality of holes with known positions and directions, each of holes being arranged to cooperate with a rod comprising a sensor; and
-    means for measuring the position of each of the sensors when the rod is inserted in the hole and the sensor is in contact with the surface of the object of interest, wherein the means for measuring comprise a magnetic reader fixed at the entry of the hole and arranged to cooperate with a magnetic railing ruler attached to the rod.

By using the frame having holes with known positions and directions, and thanks to its cooperation with the rod, the positions of the sensors, which can be used for measuring signals for imaging, can be fixed and measured that are helpful for imaging reconstruction.

In an embodiment, the system further comprises a processor for deriving the shape of the object of interest based on the positions of the plurality of sensors. When using the system according to the invention in an imaging system, for example a MIT system for patient monitoring, the position and shape information of the object of interest can be used for determining the imaging reconstruction region that leads to improve the image quality.

Another object of the invention is to develop a method of measuring an object of interest that can help derive the position and/or the shape of the object of interest.

To this end, this invention provides a method of measuring an object of interest, comprising the steps of:

-    positioning a frame around the object of interest, the frame having a plurality of holes with known positions and directions, each of holes being arranged to cooperate with a rod comprising a sensor; and

- measuring the position of each of the sensors when the rod is inserted in the hole and the sensor is attached to the surface of the object of interest.

Furthermore, the measuring step comprises the sub-steps of:

- calculating distance between the sensor and the entry of the hole by reading a magnetic signal on a magnetic reader, which is fixed at the entry of the hole and arranged to cooperate with a magnetic railing ruler attached to the rod; and
- calculating the positions of the sensors based on the distances and the known positions and directions of the holes.

This method has similar advantages as that mentioned for the system according to the invention.

In a further embodiment, the method further comprises a step of deriving the shape of the object of interest based on the positions of the plurality of sensors.

Detailed explanations and other aspects of the invention will be given below.

DESCRIPTION OF THE DRAWINGS

The above and other objects and features of the present invention will become more apparent from the following detailed description considered in connection with the accompanying drawings, in which:

Fig.1 is a schematic diagram showing an exemplary embodiment of the system in accordance with the invention;
Fig.2 is a schematic diagram showing another exemplary embodiment of the frame in accordance with the invention;
Fig.3 is a schematic diagram showing another exemplary embodiment of the frame in accordance with the invention; and

DESCRIPTION OF THE DRAWINGS

The above and other objects and features of the present invention will become more apparent from the following detailed description considered in connection with the accompanying drawings, in which:

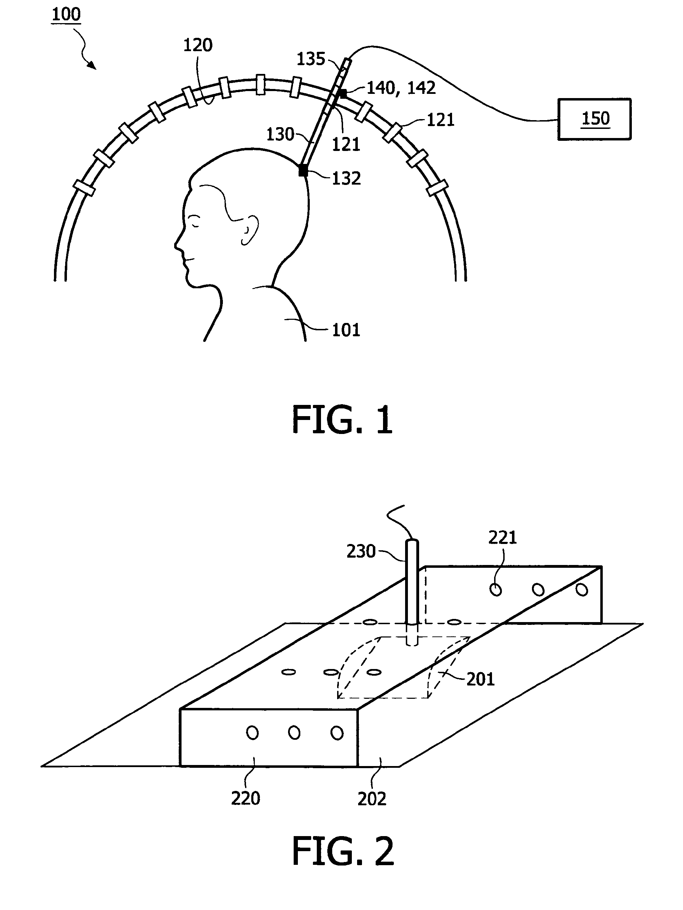

Fig.1 is a schematic diagram showing an exemplary embodiment of the system in accordance with the invention;
Fig.2 is a schematic diagram showing another exemplary embodiment of the frame in accordance with the invention;

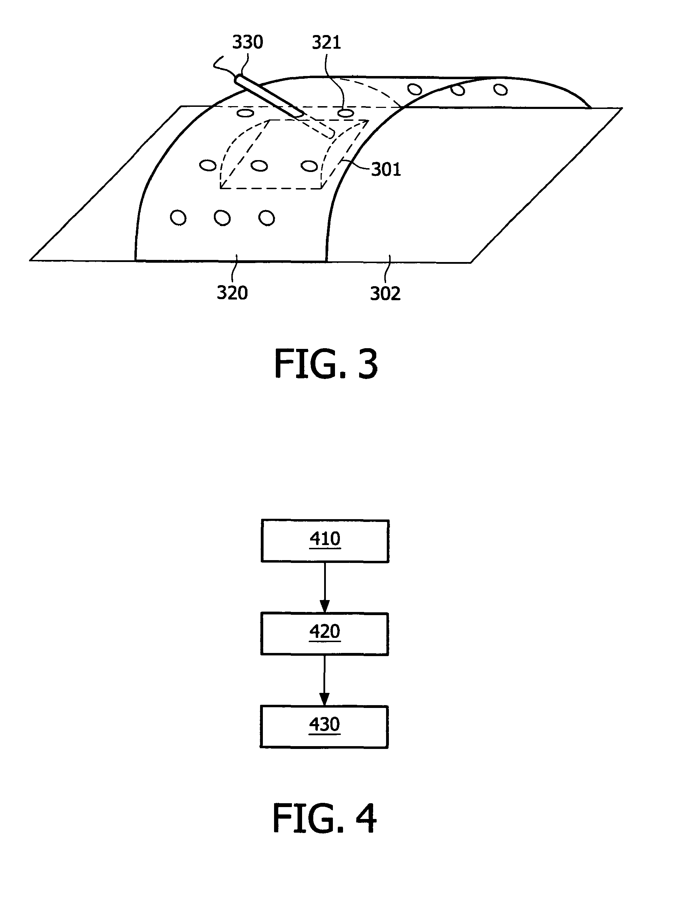

Fig.3 is a schematic diagram showing another exemplary embodiment of the frame in accordance with the invention; and
Fig.4 is a flowchart of the method in accordance with the invention.

DETAILED DESCRIPTION

Fig.1 is a schematic diagram showing an exemplary embodiment of the system 100 in accordance with the invention.

In the embodiment shown in Fig.1, the system 100 comprises a frame 120 having a plurality of open holes 121 with known positions and directions, each of holes 121 being arranged to cooperate with a rod 130 comprising a sensor 132 at one end of the rod.

In an embodiment, the frame 120 is shaped in a half sphere, and can be made of metal material, polymer, or other materials. This system particularly fits the clinical applications of brain image scanning.

The holes can be evenly or unevenly distributed on the frame, depending on the shape of the frame and/or the object to be measured. The rod can be made of metal or polymer, and the radius of the rod should fit the size of the hole, into which the rod can be inserted.

The rod comprises a sensor 132, which can be an electrode in an EIT system for measuring impedance of the object or a measurement coil in a MIT system for measuring magnetic induction signals. Alternatively, the sensor 132 can be a receiver coil in MIT system for generating excitation signals. The rod 130 can be inserted into the hole and move along the direction of the hole. The cooperation between the holes and the rods will be explained in following by explaining the way to measure the position of the sensor 132, i.e. the corresponding position of the surface of the object of interest 101 the sensor 132 is in contact with.

The system 100 further comprises means 140 for measuring the position of each of the sensors 132 when the rod 130 is inserted in the hole and the sensor 132 is in contact with the surface of the object of interest 101.

If the position of the hole is denoted by $\overline{P_i}$, the direction of the hole is denoted by a unitary vector $\overline{n}$, and the

position of the end of rod is denoted $\overline{E}_i$ (i.e., the position of the sensor 132 ), if the length of $P_iE_i$ denoted by $l_i$ is measured, the position $\overline{E}_i$ of the sensor 132 is determined by the flowing equation:

$$\vec{E}_i = \vec{P}_i + \vec{n}_i \cdot l_i \qquad\qquad [1]$$

wherein the position of the hole $\overline{P}_i$ and the direction of the hole $\overline{n}$ are known.

There are several ways to measure the length $l_i$.

According to the invention, to measure $l_i$, the means 140 comprises a magnetic reader, which is fixed at the entry of the hole and arranged to cooperate with a magnetic railing ruler attached to the rods.

Alternatively, the magnetic railing ruler may be marked on the surface of the rod. The magnetic reader can read the magnetic signal from the magnetic railing ruler when the rod is inserted in the hole and the sensor is attached on the surface of the object of interest and then the length $l_i$ can be calculated with high precision based on the magnetic signal.

By repeating the same procedure, a plurality of sensors' positions $\overline{E}_i$ , $i$ =1,2, $\cdots$, $N$ , $N$ being the number of sensors, can be determined. Because the length $l_i$ can be measured at real time, the change of sensor positions can be measured immediately.

In an embodiment, the system further comprises a processor 150 for deriving the shape of the object of interest 101 based on the positions of the plurality of sensors. The processor 150 may advantageously executes instruction codes stored in a memory (not shown) to perform this processing. As the sensors are attached to the surface of the object of interest, using the positions of the electrodes and an interpolation of their coordinates, the shape of the object of interest can be derived. The precision of the shape of the object of interest depends on the number of positions of the electrodes and the arrangement of the electrodes.

It is advantageous that the rod 130 comprises a through notch that allows the sensor 132 at one end of the rod 130 to be connected with the processor 150 by a wire through the through notch of the rod. Alternatively, the rod 130 can be hollow to allow a wire through the rod 130 to connect the sensor 132 and the processor 150. In this way, the processor can collect measurement data sensed by the sensors during monitoring and further process the measurement data for imaging.

Fig.2 is a schematic diagram showing a second exemplary embodiment of a frame 220 according to the invention.

The frame 220 is shaped as a rectangle having a plurality of holes 221 with known positions and directions. The holes can cooperate with rods 230 to measure the position of the sensors attached at one end of the each rod.

The frame 220 can be put around human body, for example, around the human chest 201 for lung function monitoring when the patient is lying on a support 202.

Fig.3 is a schematic diagram showing a third exemplary embodiment of a frame 320 according to the invention.

The frame 320 is shaped as camber having a plurality of holes321 with known positions and directions. The holes can respectively cooperate with rods 330 to measure the position of the sensors attached at one end of the each rod.

The frame 330 can be put around human body, for example, around the human breast 302 for breast monitoring when the patient is lying on a support 302.

The frame 220 is shaped as a rectangle and the frame 320 is shaped as camber. The frame 220 and 320 respectively has a plurality of holes 221, 321 with known positions and direction. The holes can respectively cooperate with rods 230 and 330 to measure the position of the sensors attached at one end of the each rod.

The frame 220 and 330 can be put around human body, for example, around the human chest 201 for lung function monitoring, or around the human breast 302 for breast monitoring when the patient is lying on a support 202, 302.

It should be noticed that the frame of the system can be designed in different shape to satisfy different clinical applications, i.e., fit the needs of measuring the object of interest 101. For example, in application of brain scan, the frame is a half sphere or round-shaped, in application of body scan, the frame can be a cylinder, and in application of breast scan, the frame can be a camber.

Fig.4 is a flowchart of the method in accordance with the invention.

According to the process in Fig.4, the method comprises a step 410 of positioning a frame around the object of interest, the frame having a plurality of holes with known positions and directions, each of holes being arranged to cooperate with a rod comprising a sensor at one end of the rod.

The method further comprises a step 420 of measuring the position of each of the sensors when the rod is inserted in the hole and the sensor is attached to the surface of the object of interest.

The measuring step 420 comprises a first sub-step of calculating distance between the sensor and the entry of the hole by reading a magnetic signal on a magnetic reader, which is fixed at the entry of the hole relative to known position of the hole and arranged to cooperate with a magnetic railing ruler attached to the rods.

The measuring step 420 further comprises a second sub-step of calculating the positions of the sensors based on the distances and the known positions and directions of the holes. The calculation can following up the method illustrated by the Equ.[1]

Equ. [1] The method may further comprise a step 430 of deriving the shape of the object of interest based on the positions of the plurality of sensors. As the sensors are attached to the surface of the object of interest, the positions of the plurality of sensors represent a plurality of positions of the surface of the object of interest, the shape of the object of interest can be derived based on the plurality of positions by using interpolations or other prior art techniques.

It should be noted that the above-mentioned system and method can be used in but not limited to imaging systems such as EIT system or MIT system. The skilled person will appreciate that the system and method provided by the invention can be used in other measuring systems, for example, in a welding system where the object to be welded has a special geometry and the geometry information is requested for matching the welding rod and torch pipe.

It should be noted that the above-mentioned embodiments illustrate rather than limit the invention and that those skilled in the art will be able to design alternative embodiments without departing from the scope of the appended claims.

**Claims**

1. A system (100) for measuring an object of interest (101), said system comprising:

   - a frame (120, 220, 320) having a plurality of holes (121, 221,321) arranged in known positions and extending in known directions, each hole being arranged to cooperate with a rod (130) comprising a sensor (132); and
   - means (140) for measuring the position of each sensor (132) when the rod is inserted in the hole (121) and the sensor (132) is in contact with the object of interest (101),
   **characterised in that** the means (140) for measuring comprise a magnetic reader (142) fixed at the entry of the hole (121) and arranged to cooperate with a magnetic railing ruler (135) attached to the rod (130).

2. A system as claimed in claim 1, further comprising a processor (150) for deriving the shape of the object of interest, based on the positions measured by said means (140) for measuring.

3. A system as claimed in claim 1, wherein the frame (120) is semi-spherical, round, cylindrical, rectangular or camber-shaped .

4. An imaging system comprising a system (100) as claimed in any one of claims 1 to 3.

5. A magnetic induction tomography system comprising a system as claimed in any one of claims I to 3.

6. An electrical impedance tomography system comprising a system as claimed in any one of claims 1 to 3.

7. A method of measuring an object of interest (101), said method comprising the steps of:

   - positioning (410) a frame (120) around the object of interest (101), the frame having a plurality of holes arranged in known positions and extending in known directions, each of the holes (121) being arranged to cooperate with a rod (130) comprising a sensor (132); and
   - measuring (420) the position of each sensor (132) when the rod (130) is inserted in the hole (121) and the sensor (132) is in contact with the object of interest (101),
   **characterised in that** the measuring step comprises the sub-steps of:
   - calculating the distance between the sensor (132) and the entry of the hole (121) by reading a signal on a magnetic reader (142), which is fixed at the entry of the hole and arranged to cooperate with a magnetic railing ruler (135) attached to the rod (130); and
   - calculating the position of the sensor (132), based on the distance and the known positions and directions of the holes.

8. A method as claimed in claim 7, further comprising a step of deriving (430) the shape of the object of interest (101), based on the positions of the plurality of sensors (132).

**Patentansprüche**

1. System zur Messung eines interessierenden Objekts (101), wobei das System Folgendes umfasst:

   - einen Rahmen (120, 220, 320) mit einer Vielzahl von Löchern (121, 221, 321), die an bekannten Positionen angeordnet sind und sich in bekannte Richtungen erstrecken, wobei jedes Loch angeordnet ist, um mit einem einen Sensor (132) umfassenden Stab (130) zusammenzuwirken; und
   - Mittel (140) zum Messen der Position jedes Sensors (132), wenn der Stab in das Loch (121) eingeführt ist und sich der Sensor (132) in Kontakt mit dem interessierenden Objekt (101) befindet,
   **dadurch gekennzeichnet, dass**
   die Mittel (140) zum Messen einen Magnetleser (142) umfassen, der am Eingang des Lochs (121) befestigt ist und angeordnet ist, um mit einem an dem Stab (130) angebrachten Magnetschienenlineal (135) zusammenzuwirken.

2. System nach Anspruch 1, weiterhin mit einem Prozessor (150) zum Ableiten der Form des interessierenden Objekts basierend auf den durch die genannten Mittel (140) zum Messen gemessenen Positionen.

3. System nach Anspruch 1, wobei der Rahmen (120) halbkugelförmig, rund, zylindrisch, rechteckig oder gewölbt ist.

4. Bildgebungssystem mit einem System (100) nach einem der Ansprüche 1 bis 3.

5. Magnetinduktionstomographiesystem mit einem System nach einem der Ansprüche 1 bis 3.

6. Elektroimpedanztomographiesystem mit einem System nach einem der Ansprüche 1 bis 3.

7. Verfahren zur Messung eines interessierenden Objekts (101), wobei das genannte Verfahren die folgenden Schritte umfasst:

   - Positionieren (410) eines Rahmens (120) um das interessierende Objekt (101), wobei der Rahmen eine Vielzahl von Löchern hat, die an bekannten Positionen angeordnet sind und sich in bekannte Richtungen erstrecken, wobei jedes der Löcher (121) angeordnet ist, um mit einem einen Sensor (132) umfassenden Stab (130) zusammenzuwirken; und
   - Messen (420) der Position jedes Sensors (132), wenn der Stab in das Loch (121) eingeführt ist und sich der Sensor (132) in Kontakt mit dem interessierenden Objekt (101) befindet,

   **dadurch gekennzeichnet, dass** der Schritt des Messens die Folgenden untergeordneten Schritte umfasst:

   - Berechnen des Abstands zwischen dem Sensor (132) und dem Eingang des Lochs (121) durch Ablesen eines Signals am Magnetleser (142), der am Eingang des Lochs befestigt ist und vorgesehen ist, um mit einem an dem Stab (130) angebrachten Magnetschienenlineal (135) zusammenzuwirken; und
   - Berechnen der Position des Sensors (132) basierend auf dem Abstand und den bekannten Positionen und Richtungen der Löcher.

8. Verfahren nach Anspruch 7, das weiterhin einen Schritt des Ableitens (430) der Form des interessierenden Objekts (101) basierend auf den Positionen der Vielzahl von Sensoren (132) umfasst.

**Revendications**

1. Système (100) pour mesurer un objet d'intérêt (101), ledit système comprenant :

   - un cadre (120, 220, 320) possédant une pluralité de trous (121, 221, 321) agencés dans des positions connues et s'étendant dans des directions connues, chaque trou étant agencé pour coopérer avec une tige (130) comprenant un capteur (132) ; et
   - des moyens (140) pour mesurer la position de chaque capteur (132) lorsque la tige est insérée dans le trou (121) et le capteur (132) est en contact avec l'objet d'intérêt (101),
   **caractérisé en ce que**
   les moyens (140) pour mesurer comprennent un lecteur magnétique (142) fixé à l'entrée du trou (121) et agencé pour coopérer avec une règle à rail magnétique (135) fixée à la tige (130).

**2.** Système selon la revendication 1, comprenant en outre un processeur (150) pour dériver la forme de l'objet d'intérêt, en fonction des positions mesurées par lesdits moyens (140) pour mesurer.

**3.** Système selon la revendication 1, dans lequel le cadre (120) est semi-sphérique, rond, cylindrique, rectangulaire ou en forme de cambrure.

**4.** Système d'imagerie comprenant un système (100) selon une quelconque des revendications 1 à 3.

**5.** Système de tomographie par induction magnétique comprenant un système selon une quelconque des revendications 1 à 3.

**6.** Système de tomographie par impédance électrique comprenant un système selon une quelconque des revendications 1 à 3.

**7.** Procédé de mesure d'un objet d'intérêt (101), ledit procédé comprenant les étapes de :

- positionnement (410) d'un cadre (120) autour de l'objet d'intérêt (101), le cadre possédant une pluralité de trous agencés dans des positions connues et s'étendant dans des directions connues, chacun des trous (121) étant agencé pour coopérer avec une tige (130) comprenant un capteur (132) ; et
- mesure (420) de la position de chaque capteur (132) lorsque la tige (130) est insérée dans le trou (121) et le capteur (132) est en contact avec l'objet d'intérêt (101), **caractérisé en ce que** l'étape de mesure comprend les sous-étapes de :
- calcule de la distance entre le capteur (132) et l'entrée du trou (121) en lisant un signal sur un lecteur magnétique (142), qui est fixé à l'entrée du trou et agencé pour coopérer avec une règle à rail magnétique (135) fixée à la tige (130) ; et
- calcule de la position du capteur (132), en fonction de la distance et des positions et directions connues des trous.

**8.** Procédé selon la revendication 7, comprenant en outre une étape de dérivation (430) de la forme de l'objet d'intérêt (101), en fonction des positions de la pluralité de capteurs (132).

# FIG. 1

# FIG. 2

# FIG. 3

# FIG. 4

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2007128952 A1
- JP 2031736 A
- JP 2031739 A